# EUROPEAN PATENT APPLICATION

(11) **EP 1 875 928 A1**
(43) Date of publication of application: **09.01.2008**
(21) Application number: 06731999.6
(22) Date of filing: 17.04.2006
(51) Int. Cl.: A61K 49/00

(54) **NOVEL METHOD OF USING TRIACETIN AND AUXILIARY AGENT FOR ULTRASONIC DIAGNOSTIC EXAMINATION**

(30) Priority: 15.04.2005 JP 2005118494
(71) Applicant: Shinshu University, Matsumoto-shi, Nagano 390-8621 (JP); National University Corporation Kagawa University, Takamatsu-shi, Kagawa 760-8521 (JP); Fushimi Pharmaceutical Company, Limited, Marugame-shi, Kagawa 763-8605 (JP)
(72) Inventor: KOYAMA, Shozo,Shinshu Uni. School of Medicin, Matsumoto-shi, Nagano 390-8621 (JP); TOKUDA, Masaaki,Faculty of Medicine, Kita-gun, Kagawa 761-0793 (JP); HORI, Yoshiyuki,Fushimi Pharmaceutical Co., Ltd., Marugame-shi, Kagawa 7638605 (JP); OHSAKA, Kazumasa,Fushimi Pharmaceutical Co., Ltd., Marugame-shi, Kagawa 763-8605 (JP)
(74) Representative: Hart Davis, Jason
(86) International application number: PCT/JP2006/308070
(87) International publication number: WO 2006/112443

(57) **Abstract**

A drug in the form of a solid preparation soluble or suspendable in water or an aqueous solution **characterized by** containing at least one biocompatible, gastric motility suppressing component such as triacetin, which is capable of transmitting ultrasonic wave to a target organ via the solution pooled in the body in a noninvasive examination (for example, an ultrasonic examination of an abdominal organ), identifying the area in which the solution exists, or clarifying the boundary of the solution and an organ in contact therewith, is employed as an auxiliary agent in the examination. In addition, triacetin can be extensively used as a gastric motility suppressor.

## Description

### TECHNICAL FIELD

The present invention relates to a stomach motility suppressant containing triacetin, a testing aid for ultrasonic imaging diagnosis, and a kit for use in ultrasonic imaging diagnosis.

### BACKGROUND ART

In order to diagnose abdominal parenchymal organs such as the liver, gallbladder, pancreas, adrenals, kidneys, and spleen, both functional and organic changes in disease must be known accurately and various means are employed to this end; they include not only fundamental tests such as history taking (covering the environment surrounding the patient, his or her background, etiology, and course of development), phonacoscopy, background investigation, physical examination, hematological test, biochemical examination of blood, and urine test but also imaging tests such as X-ray examination (with plain film or contrast medium), endoscope, ultrasonic test (US), endoscopic ultrasonography (EU), CR, MRI, and PET; these means are improving day by day. Among others, imaging diagnosis not only provides an important key to diagnostic confirmation but it is an indispensable technique for determining on which action should be taken.

Among the various imaging tests mentioned above, ultrasonic examination is a means of first choice. The reasons include not only the small burden on the person under examination but also the following: (1) it is a noninvasive examination and presents no concern for exposure to X-rays; (2) it can be repeated as many times as required; (3) observation is possible until complete satisfaction is attained; (4) it is also suitable for a wait-and-see approach; (5) the apparatus is compact and movable; (6) it can be practiced at bed side if this is required by the condition of the person under examination; (7) a real-time image can be obtained; (8) the result can be immediately accessed; (9) sections in any directions can be extracted; (10) the cost for examination is low; (11) the switch for a color Doppler needs only to be pressed to evaluate the bloodstream in a noninvasive manner; (12) and more detailed bloodstream diagnosis is possible if the examination is combined with the use of an ultrasonic contrast medium; hence, the ultrasonic examination today assumes an important position not only for screening purposes but also as a work-up method.

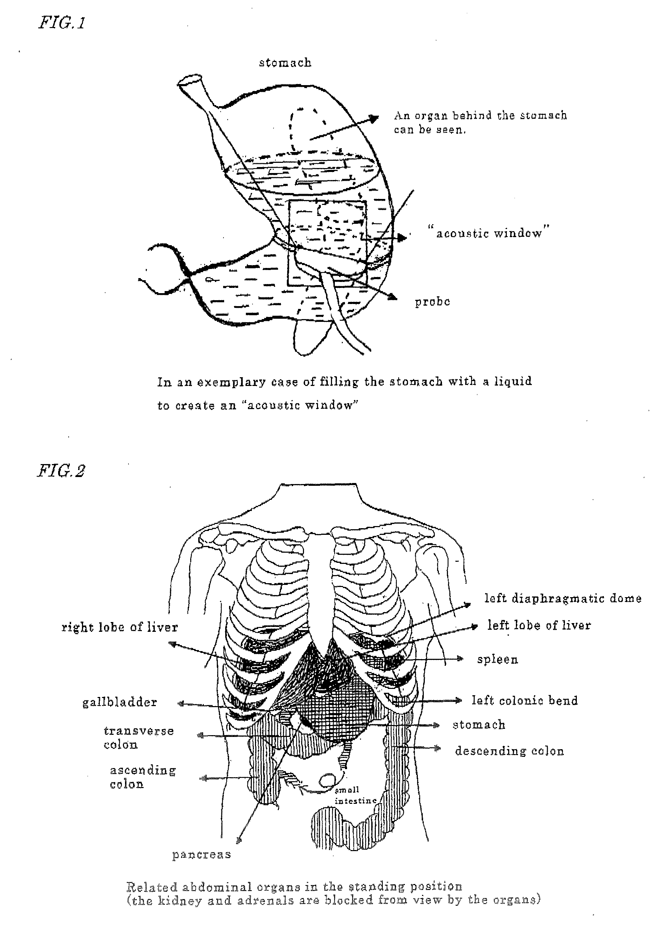

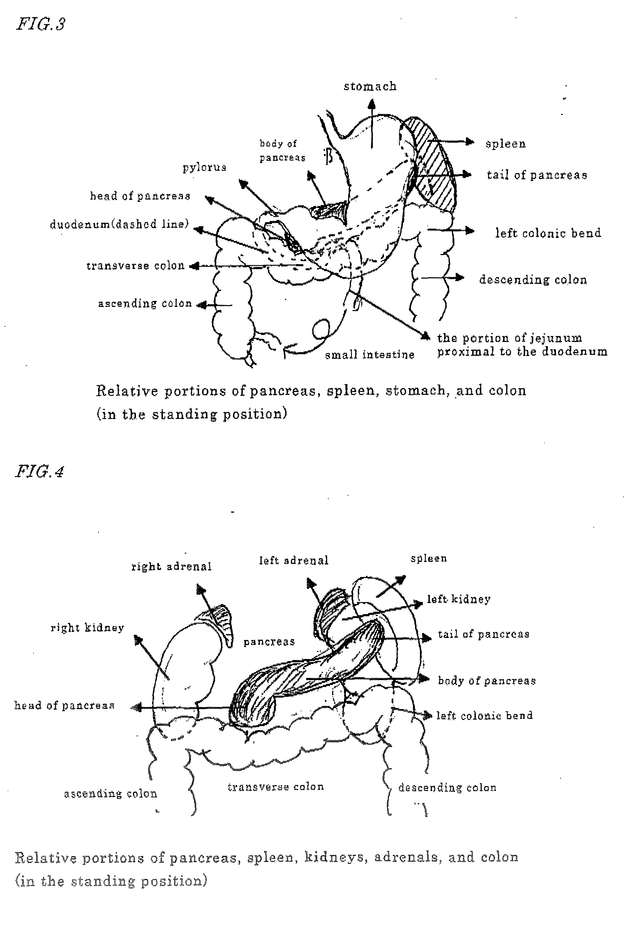

However, ultrasonic examination has one weak feature in that it tends to be influenced by the physical conditions of the person under test such as the gas in the digestive tract and obesity. Since ultrasonic waves by nature do not propagate in the air, an "acoustic window" (see FIG. 1) is created in a suitable position on the surface of the human body that will not interfere with the propagation of ultrasound, whereby the organ of interest is viewed through a two-dimensional (2D) section and recognized as a three-dimensional (3D) image by individual observers subjectively, also depending on their experience and the like; however, this always presents the problem with objectivity. The "acoustic window" cannot be created at a position that passes through an air-containing organ such as the lung or stomach although this position is preferred for observation and it is well known that the space between ribs is also unsuitable as a place to be contacted by the probe. In short, the position of the "acoustic window" is limited. Hence, it is difficult to obtain a useful image at the edge of the outward area of the left lobe of the liver, the left side of an adrenal, the superior pole and the area inward of the left kidney, and the upper portion of the spleen (under the dome of the left diaphragm) and a certain lesion sometimes fail to be noticed. In particular, the pancreas is anatomically within the diaphragmatic dome under the left ribs and behind the air-containing stomach; below the pancreas, gas is often found at the bend of the left colon, and above the pancreas are situated the lungs composed of air vesicles and air ways. Hence, it is difficult to create an "acoustic window" for viewing the pancreas, especially the tail of pancreas, and in many cases the stomach is filled with water and observation is made through the stomach lumen but water will readily flows out of the stomach. This is also physiologically well known and particularly at the time when the person under examination changes his or her position, water will readily pass through the pylorus to flow into the duodenum. Hence, it is generally held that ultrasonic examination is difficult to perform on the pancreas and other retroperitoneal organs (see FIGS. 2, 3, and 4).

To deal with this problem, a solution that stays within the stomach and which allows for efficient ultrasound propagation is described as an auxiliary agent for examination but the auxiliary agent itself will not reflect ultrasound to allow for imaging of the region where it is present. The solution that allows for efficient ultrasound propagation is a uniform solution that has no interface and is free from a refractive index gradient in it. The following are six relevant patent documents that have been published to date.

As a method of causing a contrast medium to stay within a digestive organ, Patent document I has so far proposed the use of an alginate that gives enhanced viscosity in an acidic environment or in an environment where polyvalent ions are present, and the auxiliary agent for examination is allowed to stay within the lumen by having its viscosity enhanced. Patent document 1 also states that by agitating this auxiliary agent for examination with a homogenizer and the like to generate tiny air bubbles, a suitable degree of ultrasound reflection is promoted to assist in the ultrasonic imaging diagnosis of the digestive organ.

Patent document 2 describes the auxiliary agent for examination as a contrast aid that contains at least one viscosity enhancer and insoluble polyvinyl pyrrolidine.

Patent document 3 describes a contrast medium for obtaining the image of a constituent of the body by computer tomography (CT), ultrasonography (US), magnetic resonance imaging (MRI) or the like; in particular, it describes a low-density contrast medium comprising an oil-in-water emulsion containing about 2 to about 50 vol% of oil and a specified amount of soluble salt or sugar for maintaining homeostasis in the intestines. In Example 1, the addition of gum arabic (thickener) is mentioned.

Patent document 4 describes an ultrasonic image forming composition for use in the human gastrointestinal tract, characterized by comprising an aqueous dispersion of edible inorganic particles with a size of less than 100 microns, the dispersion containing 0.1-10 g of those particles per 100 ml of water containing a dissolved hydrophilic colloid dispersant. The same document also states that the use of a viscous image former is important since it ensures rapid coating of the gastric or intestinal lining and that at the same time it contributes to avoid an unduly short or long transit time.

Patent document 5 describes a contrast imparting medium for obtaining an ultrasonic image which is characterized by comprising an aqueous solution of at least one biocompatible polymer, the biocompatible polymer being covered and/or mixed with at least one silicon-containing compound. The same document states to the effect that the polymer solution can be an aqueous solution of cellulose and that it can be used with a viscosity enhancer; cellulose is also a viscosity enhancer and, in addition, other examples of viscosity enhancer are given, such as sodium alginate and gum tragacanth (page 17, lines 14-27); it is also mentioned that the polymer is characterized by being covered and/or mixed with at least one silicon-containing compound.

Patent document 6 describes a substance having a gastric emptying time (defined as the amount of time the stomach remains distended after ingestion of a solution) of at least 20 minutes, in particular, one having an osmotic pressure of 300 millimoles/kilogram or more; the document also mentions a glyceride.

Upon closer inspection of the above-mentioned Patent documents, they may be summarized as describing the following.

According to Patent document 1, the auxiliary agent is allowed to stay within the digestive tract by virtue of viscosity; however, increased viscosity makes the auxiliary agent difficult to swallow and impairs the convenience of ultrasonic diagnosis. The difficult-to-digest viscosity enhancer has colloid osmotic pressure and induces diarrhea. If air bubbles enter into the auxiliary agent as it is swallowed, the chance of their disappearing will decrease with the increasing viscosity and the air bubbles that entered will reflect ultrasound, impairing its propagation. Patent document 1 further states that viscosity is enhanced in an acidic environment or in an environment where polyvalent ions are present; however, in these environments, the alginate becomes insoluble and forms a gel rather than a uniform solution. In other words, ultrasound is reflected or refracted and its is impaired, which is unsuitable for the creation of an "acoustic window."

According to Patent document 2, the auxiliary agent contains at least one viscosity enhancer and insoluble polyvinyl pyrrolidine and as in Patent document 1, the purpose is considered to achieve retention within the digestive tract by virtue of viscosity; however, increased viscosity makes the auxiliary agent difficult to swallow and impairs the convenience of ultrasonic diagnosis. The difficult-to-digest viscosity enhancer has colloid osmotic pressure and induces diarrhea. If air bubbles enter into the auxiliary agent as it is swallowed, the chance of their disappearing will decrease with the increasing viscosity and the air bubbles that entered will reflect ultrasound, impairing its propagation. In addition, the solution containing the insoluble polyvinyl pyrrolidine is not a uniform solution and a gel-like substance interspersed with the insoluble polyvinyl pyrrolidine will reflect or refract ultrasound and impairs its propagation, which is unsuitable for the creation of an "acoustic window."

According to Patent document 3, an oil-in-water emulsion and a specified amount of a soluble salt or sugar for maintaining the homeostasis within the intestines are used; homeostasis within the intestines means an isotonic liquid such as physiological saline, in which the oil-in-water emulsion is to be contained; the oil-in-water emulsion has an interface and as in Patent document 1, ultrasound is reflected or refracted and its propagation is impaired, which is unsuitable for the creation of an "acoustic window."

In Patent document 4, an aqueous dispersion of edible inorganic particles is used and this itself is an ultrasound reflector, Impairing the propagation of ultrasound and making it impossible to create an "acoustic window." As in Patent document 1, the purpose is considered to achieve retention within the digestive tract by virtue of viscosity; however, increased viscosity makes the testing aid difficult to swallow and impairs the convenience of ultrasonic diagnosis. The difficult-to-digest viscosity enhancer has colloid osmotic pressure and induces diarrhea. If air bubbles enter into the testing aid as it is swallowed, the chance of their disappearing will decrease with the increasing viscosity and the air bubbles that entered will reflect ultrasound, impairing its propagation, which is unsuitable for the creation of an "acoustic window."

Patent document 5 has a passage stating to the effect that the polymer solution of interest can be used with a viscosity enhancer, which is exemplified by sodium alginate, gum tragacanth and the like (page 17, lines 14-27). As in Patent document 1, the purpose is considered to achieve retention within the digestive tract by virtue of viscosity; however, increased viscosity makes the testing aid difficult to swallow and impairs the convenience of ultrasonic diagnosis. The difficult-to-digest viscosity enhancer or polymer solution has colloid osmotic pressure and induces diarrhea. If air bubbles enter into the auxiliary agent as it is swallowed, the chance of their disappearing will decrease with the increasing viscosity and the air bubbles that entered will reflect ultrasound, impairing its propagation. In addition, cellulose and the like which are used as the polymer solution do not dissolve completely but form gel-like particles, which reflect or refract ultrasound to impair its propagation. Furthermore, the use of the silicon-containing compound, which is not water-soluble, results in an interface that reflects ultrasound, impairing its propagation, which is unsuitable for the creation of an "acoustic window."

In Patent document 6, the composition is adjusted to have a gastric emptying time of at least 20 minutes and high osmotic pressure (300 millimoles/kilogram or more) so that a stomach emptying suppressing effect (already known) will be achieved; however, in order to generate this osmotic pressure, a water-soluble substance is required but the difficult-to-digest polydextrose solution and the like that are mentioned in the document have colloid osmotic pressure together with viscosity and this viscosity makes the composition difficult to swallow, impairs the convenience of ultrasonic diagnosis, and induces diarrhea on account of the colloid osmotic pressure. If air bubbles enter into the auxiliary agent as it is swallowed, the chance of their disappearing will decrease with the increasing viscosity and the air bubbles that entered will reflect ultrasound, impairing its propagation. In addition, the glyceride and the like that are described in the document usually form an interface in the aqueous solution to reflect ultrasound and impair its propagation, which is unsuitable for the creation of an "acoustic window."

Hence, preparations that intend ultrasonic propagation with a view to creating an "acoustic are uniform solutions that have no interface and which are free from a refractive index gradient in the solution. The conditions that should be avoided by such preparations are listed below:
(1) their viscosity should not be high (because high viscosity makes the preparation difficult to swallow, impairs the convenience of ultrasonic examination, and the more viscous the liquid is, the smaller the chance of air bubbles to disappear);
(2) an oil-in-water emulsion and the dispersion of oil droplets should be avoided (because they reflect or refract ultrasound);
(3) insoluble particles and gel-like particles should be avoided (because they reflect or refract ultrasound);
(4) a difficult-t-digest water-soluble polymer should be avoided (because it gengerates a colloid osmotic pressure and induces diarrhea.)
   Therefore, drugs for use in ultrasonic examination and the like that do not depend on viscosity or non-ingestible water-soluble polymers for staying in the stomach are strongly desired and the present inventors launched efforts to develop triacetin preparations as drugs that satisfy all of the conditions listed above.

In treatments such as preliminary ones that precede examination of the stomach with X-rays or an endoscope, butylscopolamine bromide, glucagons and the are commonly applied by injection. Butylscopolamine bromide acts on parasympathetic ganglions and impose a great burden on the person under examination; glucagons are known to have such a serious side effect that the blood glucose level is lowered on account of a rebounding action; hence, other non-invasive gastric motility suppressors are currently desired.
PATENT DOCUMENT 1: JP 10-306042 A
PATENT DOCUMENT 2: JP 10-502382 A
PATENT DOCUMENT 3: JP 62-289531 A
PATENT DOCUMENT 4: JP 5-92930 A
PATENT DOCUMENT 5: JP 2001-500469 A
PATENT DOCUMENT 6: Japanese Patent No. 2918692

### DISCLOSURE OF THE INVENTION

### PROBLEM TO BE SOLVED BY THE INVENTION

The present invention aims to provide a gastric motility suppressor that is noninvasive to persons under examination.

In radiographic examination of the stomach, it is required to eliminate gastric motility and drugs currently used to that end include parasympatholytic agents such as powerful butylscopolamine bromide, as well as glucagons which is also a powerful drug. Butylscopolamine bromide causes various side effects including shocks (e.g. nausea, vomiting, chill, pallor of the skin, and lowering of blood pressure), eyes (accommodative disorder), digestive organs (water thirst, nausea, or vomiting), urinary organs (dysuria), psychoneurotic effects (headache, heaviness of the head, sleepiness, and vertigo), circulatory organs (palpitation), hypersensitivity (rash), and others (hot flush); glucagons causes many serious side effects including shocks, anaphylactic shocks (initial symptoms: discomfort, pallor of the face, lowering of blood pressure, etc.), hypoglycemic symptoms such as malaise, somnolence and pallor of the face, digestive organs (feeling of sickness, stomachache, borborygmus, vomiting, and diarrhea), blood (an increase in the number of leukocytes, and variation of leukocyte fractions), vascular system (palpitation and lowering of blood pressure), liver (elevation of serum bilirubin), sugar metabolism (elevation of blood glucose level and sugar in the urine), lipid metabolism (elevated TG), and others (bad complexion, perspiration, heat, redness, vertigo, lassitude, hot flash, psychroesthesia, elevated LDH, elevated serum K, lowered serum K, headache, elevated serum inorganic phosphorus, and occult blood in the urine); despite these side effects, butylscopolamine bromide and glucagons are being used, simply due to the absence of their alternatives, but such alternatives are strongly demanded.

In addition, if water is used to create an ultrasonic propagating acoustic window in the stomach whereas one of the drugs mentioned above is used to suppress gastric motility, convenience which is the greatest advantage of ultrasonic examination is impaired.

In ultrasonic examination, the stomach which is a hollow organ contains the air in it and is unable to propagate ultrasound, so even if the person under examination is allowed to drink water to fill the stomach with it, the water cannot be retained in the stomach if the person is in the supine position or in the right lateral decubitus position, extreme difficulty has been In the case where the pancreas is to be examined. Under these circumstances, it was hoped that liquids capable of staying in the stomach would be developed and it was desired to enable ultrasonic examination of the bowel with an auxiliary agent that would suppress the gastric emptying motion temporarily so that the liquid could be retained in the stomach without interfering with the propagation of ultrasound.

With this auxiliary agent, examination can be made through the stomach and image information can be obtained from new angles while observing the liver, pancreas, adrenals, kidneys or spleen to improve the diagnostic yield. In particular, even better efficiency is obtained when observing the tail of the pancreas which is in such an anatomical relative position that it is preferably observed through the stomach.

### MEANS FOR SOLVING THE PROBLEM

The present inventors had made studies on a gastric motility suppressing comoponent that would be noninvasive to persons under examination; they have invented something that causes less side effects and which does not depend on viscosity as a material's property for its performance, thereby contributing to radiographic examination and ultrasonic examination of the stomach.

The present invention is described below with retention in the stomach taken as an example.

The present invention relates to a solid preparation, an aqueous solution or a suspending agent that comprise a component which, when they are administered as an aqueous solution or a suspension for use, stays in the stomach for as long as is necessary for examination and closes the pylorus temporarily and which can permit ultrasonic examination of the bowel with the stomach being used as an acoustic window.

Thus, the present invention provides an auxiliary agent in the form of a solid preparation soluble or suspendable in water or an aqueous solution, characterized by containing at least one biocompatible component capable of suppressing gastric motility, which is capable of transmitting ultrasonic wave to a target organ via the solution pooled in the body in a noninvasive examination (for example, an ultrasonic examination of an abdominal organ), identifying the area in which the solution exists, or clarifying the boundary of the solution and an organ in contact therewith.

The mechanism of suppressing gastric motility by means of chemical substances remains, as of today, to be clearly unraveled, except that it is generally known that gastric motility is suppressed by the presence of long-chain monobasic organic acids (fatty acids), glycerin esters (a kind of glycerides) of long-chain monobasic organic acids, amino acids, or peptides in the duodenum. However, it has become clear in the present invention that this effect is also exhibited by triacetin which is a triglyceride of a short-chain monobasic organic (acetic acid).

If auxiliary agent for examination in ultrasonic imaging diagnosis is in the state of an emulsion, the oil-water interface reflect ultrasound and affect its propagation. However, the-gastric motility suppressing component to be used in the present invention does not form an emulsion and hence will not be an impediment to ultrasound propagation. For instance, triacetin described in the present invention can be dissolved in water in an amount of about 7% at ordinary temperature without forming an emulsion and this concentration is sufficient for suppressing gastric motility.

In order to form an emulsion, a suitable surfactant is required and since the lipophylic groups face inward to enclose the fat, the site at the duodenum (the pyloric outlet) where the gastric motility suppressing action will develop its effect cannot be acted upon. This would be why the gastric motility suppressing action is not readily exhibited when it is in the state of an emulsion. Thus, among the gastric motility suppressing components described in the present invention, those which dissolve in water are dissolved as such and those which will not are first orally administered as such and then a suitable amount of a solution such as water that will not be an impediment to propagation is administered, whereby a liquid that is necessary for ultrasonic diagnosis is ensured in the stomach.

The present invention also provides a kit comprising an auxiliary agent for examination capable of transmitting ultrasonic wave to an organ, characterized by containing at least one biocompatible, gastric motility suppressing component, an instruction manual for administering the auxiliary agent for examination, and packaging supplies.

The present invention further provides a gastric mobility suppressor containing triacetin as an active ingredient.

The present invention still further provides use of triacetin in the manufacture of pharmaceuticals for suppressing gastric motility.

In addition, the present invention provides a method of suppressing gastric motility comprising administering an effective amount of triacetin.

Further in addition, the present invention provides a kit comprising a pharmaceutical composition comprising triacetin, an instruction manual describing the method of using the pharmaceutical composition, and packaging supplies.

### EFFECTS OF THE INVENTION

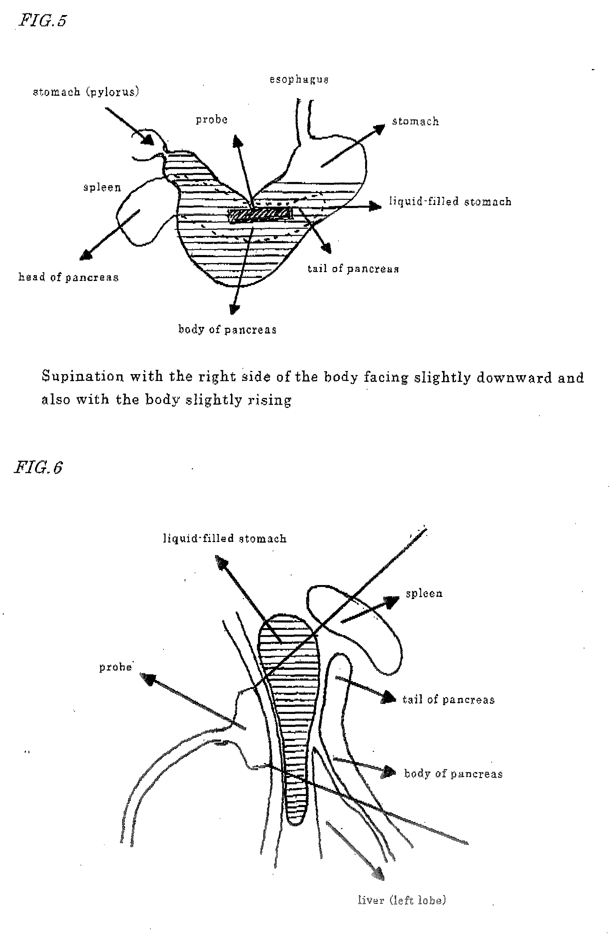

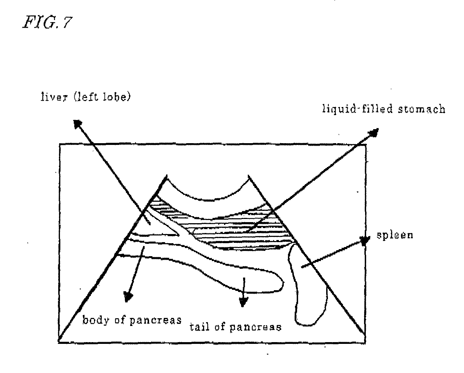

According to the present invention, the pylorus is temporarily closed and a liquid is allowed to stay in the stomach, whereby the head and tail of the pancreas the images of which are particularly difficult to acquire can be easily imaged by observation through the stomach (FIGS. 5, 6, and 7).

Further, by means of observation through the stomach according to the present invention, it also becomes possible to obtain image information from new angles for the edge of the outward area of the left lobe of the liver, the left side of an adrenal, the superior pole and the area inward region of the left kidney, and the upper portion of the spleen (under the dome of the left diaphragm), whereupon these areas can be observed with ease.

As a further advantage, it becomes possible to reduce the mental and physical burdens that may be imposed on the person under examination by treatments such as preliminary ones that precede a radiographic or endoscopic examination of the stomach.

### BRIEF DESCRIPTION OF THE DRAWINGS

[FIG. 1] FIG. 1 is a diagram illustrating the state of a case where the stomach is filled with a liquid to create an "acoustic window."
[FIG. 2] FIG. 2 is a diagram showing the relative positions of organs in the bowel.
[FIG. 3] FIG. 3 is a diagram showing the relative positions of the pancreas, spleen, stomach and colon after the liver has been removed.
[FIG. 4] FIG. 4 is a diagram showing the relative positions of the pancreas, spleen, kidneys and colon after the liver and stomach have been removed.
[FIG. 5] FIG. 5 Is a diagram showing an "acoustic window" in the body of stomach in the supine position.
[FIG. 6] FIG. 6 is a schematic sectional view showing the case of observing the tail of pancreas through the stomach.
[FIG. 7] FIG. 7 is a diagram showing image information (B mode image) in the case of observing the tail of pancreas through the stomach.
[FIG. 8] FIG. 8 is a schematic diagram of a system for evaluating the suppression of gastric emptying motility.
[FIG. 9] FIG. 9 is a graph showing the time course of the percent emptying after administering triacetin.
[FIG. 10] FIG. 10 is a graph showing comparison for period (a) on FIG. 9.
[FIG. 11] FIG. 11 is a graph showing comparison for period (b) on FIG. 9.
[FIG. 12] FIG. 12 is a graph showing the time course of the percent emptying after administering olive oil.
[FIG. 13] FIG. 13 is a graph showing the time course of the percent emptying after administering stearic acid.
[FIG. 14] FIG. 14 is a schematic diagram of a system for evaluating the measurement of gastric contractile motion.
[FIG. 15] FIG. 15 shows data from the measurement of fasting gastric motion pattern by SGT.
[FIG. 16] FIG. 16 shows data from the measurement of muscle contraction by SGT upon administration of distilled water (F), triacetin (D), or olive oil (E).

### BEST MODES FOR CARRYING OUT THE INVENTION

As the drug of the present invention, triacetin can be orally administered in either one of the following dosage forms, liquid, powder, granule, tablet, and capsule. One way to produce capsules may be as follows: a gelatin coating solution which has been heated at about 60°C to be in the state of a sol is chilled to become a gel on the surface of a casting drum in a rotary filling machine to form a gelatin sheet about 1 mm thick; two gelatin sheets, right and left, are fed along a wedge-shaped segment between two die rolls and closed at the lower part by thermal compression to mold a capsule preform, into which triacetin is forced by means of a pump while at the same time the upper part of each gelatin sheet is compressed to mold a capsule; subsequently, the is dried by blowing low-humidity air into a rotating tumbler/dryer until its shape is set; thereafter, the capsule is further dried in a low-humidity drying chamber over a prolonged period.

The auxiliary agent for examination of the present invention contains, in addition to the gastric motility suppressing component, none or at least one of an amino acid, a surfactant, a soluble protein, a polymeric compound, a silicon-containing compound, an organic acid, a polyhydric alcohol, and a bitterness suppressing component, provided that they have biocompatibility.

The gastric motility suppressing component is orally administered either as such or in the form of a tablet or capsule in an amount in the range of 0.001-20 g and, subsequently, a solution that will not interfere with the propagation of ultrasound is orally administered in an amount of about 300 mL. Alternatively, the gastric motility suppressing component is dissolved in the solution that will not interfere with the propagation of ultrasound or granules are prepared using a substance that will not interfere with the propagation of ultrasound and, prior to administration, are dissolved in the solution that will not interfere with the propagation of ultrasound; either type of solution may be orally administered in an amount of about 300 mL. The above-mentioned solution that will not interfere with the propagation of ultrasound may be water or a polyhydric alcohol, either or in admixture, and it may contain one or more of an amino acid, a surfactant, a soluble protein, a polymeric compound, a silicon-containing compound, an organic acid, and a polyhydric alcohol, provided that they have biocompatibility.

In addition, in a treatment such as a preliminary one that precedes radiographic or endoscopic examination of the stomach, triacetin may be administered in one of the aforementioned dosage forms in an amount of 0.001-20 g, whereupon the gastric motility suppressing effect can hopefully be obtained. Desirably, a dosage of about 3 g per administration would be suitable.

In the present invention, "biocompatibility" means such a property that no adverse effects will be exerted on the tissue of the body, body fluids or any other organs in a specified use.

In the present invention, the "gastric motility suppressing component" means a substance that slows down the emptying of the gastric contents from the stomach by activating the nerve- and endocrine-mediated mechanisms when the gastric contents move from the stomach to the duodenum as the contraction of the gastric vestibule allows them to be emptied in small increments by passing through the pylorus into the duodenal cap. Examples include glycerin fatty acid esters, vegetable oils, glyceryl ethers, glycerophospholipids, sphingophospholipids, sphingoglycolipids, glyceroglycolipids, and mixtures of two or more of these compounds. To state more specifically, glycerin fatty acid esters include mono- or di- or tri-esters of glycerin and saturated or unsaturated fatty acids having 2-18 carbon atoms: vegetable oils include almond oil, olive oil, cacao oil, wheat germ oil, sesame oil, safflower oil, soybean oil, camellia oil, corn oil, rapeseed oil, sun flower oil, palm oil, and cottonseed oil: glyceryl ethers include mono- or di- or tri-ethers, shown by the following formulas, of glycerin and saturated or unsatured alkyl groups having 2-18 carbon atoms:

[Chemical Formula 1]

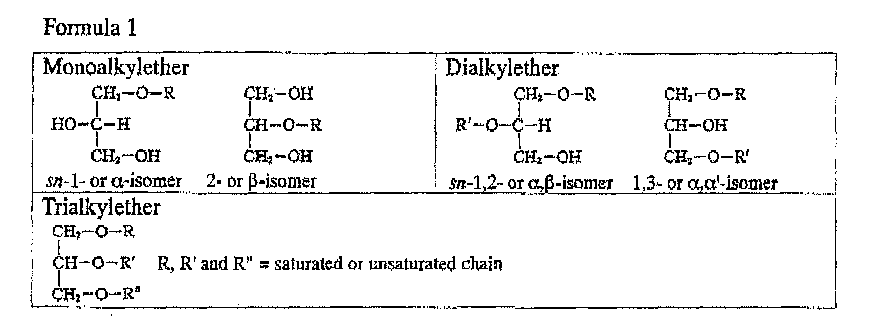

or esters of those ethers and saturated or unsaturated fatty acids having 2-18 carbon atoms, as shown by the following formulas:

[Chemical Formula 2]

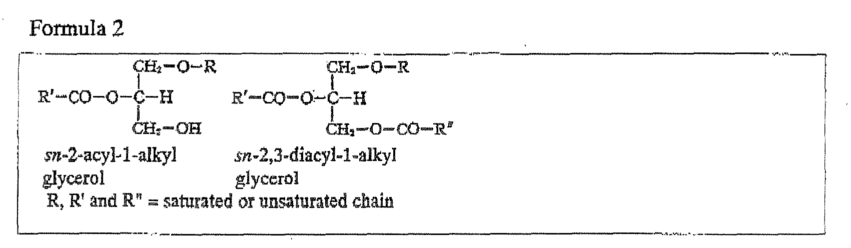

include mono- or of glycerin and or unsaturated fatty acids having 2-18 carbon atoms, in turn are esterified with phosphoric acid, aminoalcohol, or a polyol group, as shown by the following formulas:

[Chemical Formula 3]

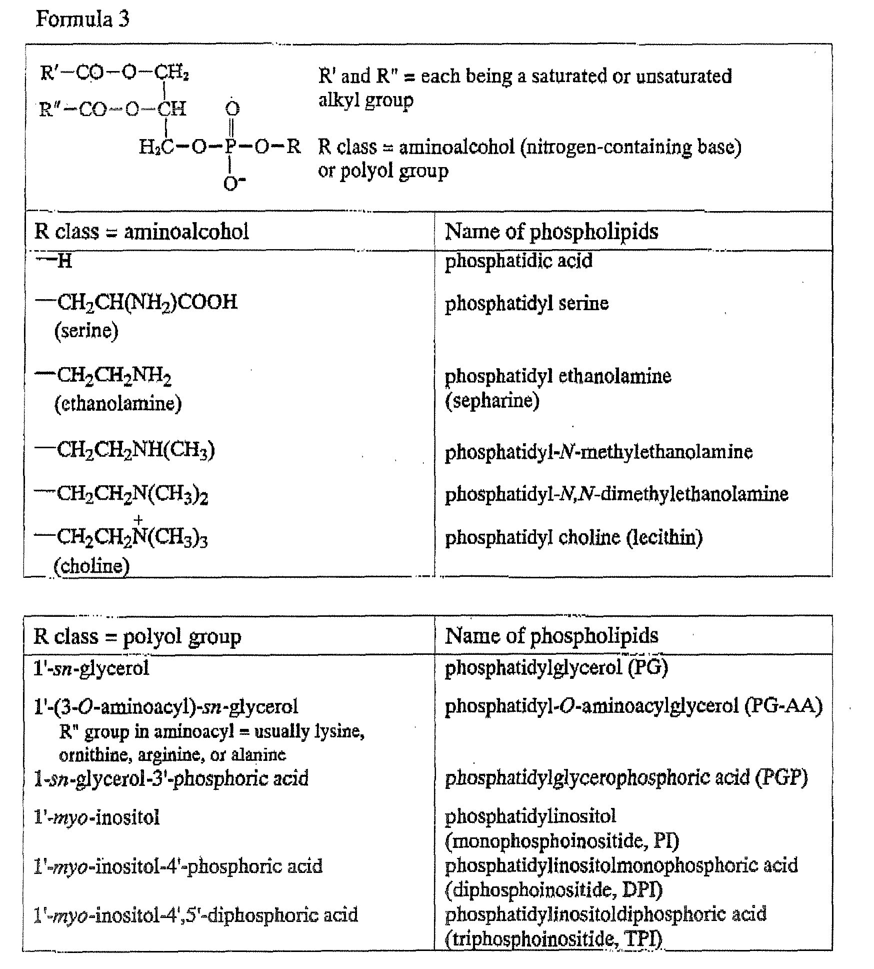

sphingophospholipids include N-acyl-trans-4. sphingenlne-1-phosphorylcholine, N-acylsphingenine-1-phosphorylcholine, ceramide aminoethyl phosphonate (ceramide ciliatine), and ceramide-N-methylaminoethyl phosphonate; and glyceroglycolipids include monogalactosyl diglylceride (MGD), digalactosyl diglyceride (DGD), and galactosylglucosyl diglyceride; however, these are not the sole examples that can be used. Among these, any two or more species may be used in combination.

The glycerin fatty acid esters as the gastric motility suppressing component are mono- or di- or tri-esters with fatty acids, in particular, saturated or unsaturated fatty acids having 2-18 carbon atoms; any vegetable oil has the ability to suppress gastric emptying motility whether it contains triacetin having a small number of carbon atoms (2 carbon atoms) or tri-n-caprylin having a medium number of carbon atoms (8 carbon atoms) or a fatty acid component having a large number of carbon atoms (18 carbon atoms). In addition, surfactants that are required to suspend those glycerin fatty acid esters in water are effective in suppressing gastric emptying motility. Amino acids, soluble proteins, polymeric compounds, organic acids, fatty acids and polyhydric alcohols are effective in stabilizing the suspension of glycerin fatty acid esters and hence assist in suppressing gastric emptying motility. Silicon-containing compounds sometimes defoam the gastric emptying motility suppressing aqueous solution to thereby prevent random reflection of ultrasound.

In the present invention, the amino acid means a protein hydrolyzate, which may be exemplified by sodium L-aspartate, DL-alanine, glycine, L-glutamic acid hydrochloride, L-arginine L-glutamate, L-lysine L-glutamate, and L-cysteine, which are by no means the sole examples that can be used. Among these, any two or more species may be used in combination.

In the present invention, the surfactant means such that a hydroxyl group or a hydrophilic group having an ether bond and a hydrophobic group from a fatty acid are present in one molecule to lower surface tension and examples include, but are not limited to, acetylglycerin fatty acid esters, propylene glycol monostearate, sucrose fatty acid esters, polyoxyl stearate, yolk lecithin, soybean lecithin, sorbitan sesquioleate, sorbitan fatty acid esters, sorbitan trioleate, polyoxyethylene sorbitan trioleate, sorbitan tristearate, polyoxyethylene sorbitan tristearate, ascorbic acid palmitate, partially hydrogenated soyben phospholipids, propylene glycol fatty acid esters, polyoxyethylene hardened castor oil, polyoxyethylene castor oil, polysorbate, glycerin monooleate, sorbitan monooleate, polyethylene glycol monooleate, polyoxyethylene sorbitan monooleate, ethylene glycol monostearate, propylene glycol monostearate, polyoxyethylene glycerin monostearate, polyoxyethylene sorbitan monostearate, sorbitan monopalmitate, glycerin monomyristate, sorbitan monolaurate, polyethylene glycol monolaurate, polyoxyethylene sorbitol monolaurate, and lauromacrogol. Among these, any two or more species may be used in combination.

In the present invention, the soluble protein means protein's partial hydrolyzates and salts thereof and examples include, but are not limited to, hydrolyzed gelatin, casein sodium, and casein peptone. Among these, any two or more species may be used in combination.

In the present invention, the polymeric compound means polymerization products of monomers having a hydroxyl group, a group having an ether bond, a group having a carboxyl group, or a group having amide or lactam and examples include, but are not limited to, arginic acid, sodium arginate, propylene glycol arginic acid ester, alpha starch, carrageenan, karaya gum powder, carboxyvinyl polymer, carboxymethyl cellulose, carboxymethyl starch sodium, carmellose potassium, carmellose sodium, *kambai-ko*, xanthan gum, guar gum, cross-carmellose sodium, cross-pohidone, succinated gelatin, copolypidon, chondroitin sodium sulfate, cellulose acetate, dextran, soluble starch, tragacanth, semi-digested starch, microcrystalline cellulose, hydroxyethyl cellulose, hydroxyethyl methylcellulose, hydroxypropyl starch, hydroxypropyl cellulose, hydroxypropyl methylcellulose, partial alpha starch, pullulan, polyvinylpyrrolidone, poly(sodium acrylate), partially neutralized poly(acrylic acid), polyvinyl alcohl (completely saponified), polyvinyl alcohol (partially saponified), polypropylene glycol, polyethylene glycol, fumaric acid, propionic acid, maleic acid, and malonic acid. Among these, any two or more species may be used in combination.

In the present invention, the silicon-containing compound means a compound having repeated silicon-oxygen bonds and examples include, but are not limited to, dimethyl polysiloxane (methyl polysiloxane), dimethyl polysiloxane/silicon dioxide mixture, and dimethyl siloxane/methyl(polyloxyethylene)siloxane copolymer. Among these, any two or more species may be used in combination.

In the present invention, the fatty acid means long-chain monobasic organic acids or salts thereof as a constituent of the glycerin fatty acid ester which is the gastric motility suppressing component and examples include, but are not limited to, isostearic acid, sodium caprylate, potassium stearate, sodium stearate, oleic acid, linoleic acid, linolenic acid, palmitic acid, and myristic acid. Among these, any two or more species may be used in combination.

In the present invention, the organic acid means monomolecular compounds having a carboxyl group other than the aforementioned fatty acids or salts of such monomolecular compounds and examples include, but are no limited to, adipic acid, sodium dihydrogen citrate, disodium citrate, gluconic acid, succinic acid, monosodium succinate, disodium succinate large hydrate, tartaric acid, sodium DL-tartrate, sodium L-tartrate, potassium sodium tartrate, potassium sodium tartrate, and sodium dl-pyrrolidonecarboxylate. Among these, any two or more species may be used in combination. In the present invention, the polyhydric alcohol means organic compounds, except saccharides, that have two or more hydroxyl groups in one molecule and examples include, but are not to, propylene glycol, as well as glycerin and glycol. Among these, any two or more species may be used in combination.

In the present invention, the bitterness suppressing component means a component that suppresses the bitterness of triacetin and examples include, but are not limited to, DL-alanine, disodium 5'-inosinate, erythritol, carbachol, dl-camphor, disodium 5'-guanylate, glycine, glycyrrhizinic acid, trisodium glycyrrhizinate, diammonium glycyrrhizinate, dipotassium glycyrrhizinate, disodium glycyrrhizinate, monoammonium glycyrrhizinate, glucono-δ-lactone, β-cyclodextrin, potassium hydrogen tartrate, tannic acid, middle-chain fatty acid triglycerides, tetrasodium pyrophosphate, balsam of Peru, povidone, lipoproteins, and dl-menthol. Among these, any two or more species may be used in combination.

In the present invention, the concentration for dissolution in water is desirably such that dissolution is achieved mainly at liquid temperatures from 0°C to 90°C.

If a preparation comprising the above-mentioned components is allowed to stay in the stomach of the human body for at least about 15 minutes, a probe may be brought into contact with the stomach from the front of its anterior body so that a target organ can be observed through the retained water.

The preparation can also be used as an auxiliary agent for examination with an ultrasound endoscope that is fitted at the tip with an ultrasonic transducer for sending or receiving an echo.

In the case of an auxiliary agent for ultrasonic examination using water-soluble gastric motility suppressing components, an amount suitable for creating an "acoustic window" in the stomach is administered perorally to the person under examination. The dosage varies from person to person depending on the size of his or her stomach and the like but a suitable dosage is such that the total volume including the solution that will not interfere with the propagation of ultrasound is from approximately 50 mL to 1000 mL. The auxiliary agent for examination that is dosed per examination may contain 0.001 g to 20 g of the gastric motility suppressing component, and if the agent is to be used as a gastric motility suppressor containing triacetin as an active ingredient, it may be administered in an amount of 0.001 g to 20 g. The kit of the auxiliary agent for examination according to the present invention has an instruction manual describing the proper method of use, precautions and the like, as well as a pharmaceutically appropriate container; if desired, it may have an auxiliary device for assisting in administration to the person under examination.

From the foregoing explanation, a skilled artisan can easily infer non-medicinal goods that contain triacetin in anticipation of the gastric motility suppressing effect. Examples of such goods include triacetin-containing functional foods. Such functional foods may take various forms including drinks, confectioneries, powdered capsule foods, tablet foods, etc.
The present is hereinafter in greater detail by to examples, which are no means intended to limit the of the present invention.

### EXAMPLES

The following are examples for the method of making preparations.

### Example 1 (Triacetin (0.5%))

To 200 mL of water under agitation with a magnetic stirrer, 1.5 g of triacetin, 0.24 g of citric acid and 1.74 g of trisodium citrate, both as a pH modifier, and 3 g of glycerin as a sweetener were added, followed by heating at 80°C.

Subsequently, 0.012 g of ethyl paraoxybenzoate and 0.027 g of butyl paraoxybenzoate that had been preliminarily dissolved in 0.45 g of propylene glycol were added; after cooling, a preliminarily prepared ethanol solution of 20% 1-menthol was added in such an amount that 0.006 g of 1-menthol would be incorporated and water was added to make a volume of 300 mL. As a result, a colorless and clear preparation was completed.

Thus, the completed preparation contains the following components (A) to (I) in 300 mL of water.

Since triacetin tastes bitter, additives and a method for eliminating the bitterness may optionally be used.
(A) triacetin: 1.5 g
(B) buffer: citric acid: 0.24 g
(C) buffer: trisodium citrate: 1.74 g
(D) sweetener: glycerin: 3 g
(E) solvent promoter: propylene glycol: 0.45 g
(F) antiseptic: ethyl paraoxybenzoate: 0.012 g
(G) antiseptic: butyl paraoxybenzoate: 0.027 g
(H) flavoring agent: 1-menthol: 0.006 g
(I) solvent promoter: ethanol: q.s.

### Example 2 (Triacetin (0.5%))

To 200 mL of water under agitation with a magnetic stirrer, 1.5 g of triacetin, 0.24 g of citric acid and 1.74 g of trisodium citrate, both as a pH modifier, and 0.03 g of saccharin sodium as a sweetener were added, followed by heating at 80 °C.

Subsequently, 0.012 g of ethyl paraoxybenzoate and 0.027 g of butyl paraoxybenzoate that had been preliminarily dissolved in 0.45 g of propylene glycol were added; after cooling, 3 g of glycine and a preliminarily prepared ethanol solution of 20% 1-menthol were added in such an amount that 0.006 g of 1-menthol would be incorporated and water was added to make a volume of 300 mL. As a result, a colorless and clear preparation was completed.

Thus, the completed preparation contains the following components (A) to (J) in 300 mL of water.
(A) triacetin: 1.5 g
(B) buffer: citric acid: 0.24 g
(C) buffer: trisodium citrate: 1.74 g
(D) sweetener: saccharin sodium: 0.03 g
(E) solvent promoter: propylene glycol: 0.45 g
(F) antiseptic: ethyl paraoxybenzoate: 0.012 g
(G) antiseptic: butyl paraoxybenzoate: 0.027 g
(H) bitterness suppressor: glycine: 3 g
(I) flavoring agent; 1-menthol: 0 006 g
(J) solvent promoter: ethanol: q.s.

### Example 3 (Triacetin (10%))

Thirty grams of triacetin and 4.5 g of polyoxyethylene(30) hardened castor oil (Nikko Chemicals Co., Ltd.: NIKKOL HCO-30) were mixed to give the indicated proportions and agitated with a high-performance stirrer/disperser, ULTRA-TARAX (product of IKA JAPAN CO., LTD.) to yield a liquid mixture.

To 200 mL of water heated at about 70°C, 0.6 g of carmellose sodium (CELOGEN F-SC of DAI-ICHI KOGYO SEIYAKU CO., LTD.) was added under agitation with a magnetic stirrer to form a solution. After cooling to room temperature, 0.24 g of citric acid and 1.74 g of trisodium citrate, both as a pH modifier, and 0.03 g of saccharin sodium as a sweetener were added, followed by heating at 80°C.

Then, 0.012 g of ethyl paraoxybenzoate and 0.027 g of butyl paraoxybenzoate that had been preliminarily dissolved in 0.45 g of propylene glycol were added; after thorough agitation, the temperature of the liquid was lowered to about 50°C.

Subsequently, the whole quantity of the aforementioned liquid mixture was added and after agitation, water was added to make a volume of 300 mL, which was emulsified with a high-performance stirrer/disperser (ULTRA-TARAX, product of IKA JAPAN CO., LTD.)

Thus, the completed preparation contains the following components (A) to (J) in 300 mL of water.
(A) triacetin; 30 g
(B) polyoxyethylene(30) hardened castor oil: 4.5 g
(C) carmellose sodium: 0.6 g
(D) citric acid: 0.24 g
(E) trisodium citrate: 1.74 g
(F) sweetener: saccharin sodium: 0.03 g
(G) propylene glycol: 0.45 g
(H) ethyl paraoxybenzoate: 0.012 g
(I) butyl paraoxybenzoate: 0.027 g

### Example 4 (Triacetin (granules))

A silicone resin (0.83 g), 0.216 g of polyoxyethylene sorbitan monooleate (NIKKOL: TO-10M) and 0.089 g of sorbitan sesquioleate (NIKKOL:SO-15R) were suspended in 100 g of IPA and after adding 1 g of PVP, the latter was completely dissolved (silicone solution).

Subsequently, a high-speed kneader (SEISHIN ENTERPRISE CO., LTD., Model: New Gramachine (NG-200)) was charged with 824 g of fructose preliminarily ground with an atomizer, the whole quantity of the separately prepared silicone solution, and 165 g of triacetin in the order written, and the contents were mixed. The mixture was extruded through a blade, basket-type extrusion granulator (KIKUSUI SEISAKUSHO LTD., Model: RG-5) and fed into a spheroidizer (Fuji Paudal Co., Ltd. Model: 0-230).

The prepared granules were transferred into a vat, where they were dried at 50°C for 2 hours; thereafter, the granules were sifted on two sieves, one with openings of 355 and the other 840 µm, and the that passed through the 840-µm openings but which were on the 355-µm openings were as the desired preparation (granular).

The mode of administration should finally be determined by considering various factors such as the concentration of triacetin and dose; in one example, deaerated water may be added to 18 g of the above produced preparation to make a volume of 300 g (1% triacetin in water) and the whole quantity of the aqueous solution is administered perorally.

### Test 1: Comparing the Viscosities of Triacetin, Water, and Viscosity Enhancing Polysaccharide

Using water (ion-exchanged water), an aqueous solution of triacetin, and an aqueous solution of sodium alginate which is a typical viscosity enhancing polysaccharide (KIMICA Corporation, Grade: IL-2), viscosity comparison was made. Viscosity was measured with a Brookfield viscometer (product of TOKIMEC INC., Model BM).

As the result, triacetin was verified to be considerably less effective in viscosity enhancement as compared with sodium alginate.

**[Table 1]**

| Sample | Viscosity (mPa.s) |
|---|---|
| Water | 10.0 |
| Triacetin (0.2 %) | 10.1 |
| Triacetin (1.0 %) | 10.8 |
| Triacetin (5.0 %) | 16.7 |

**[Table 2]**

| Sample | Viscosity (mPa.s) |
|---|---|
| Sodium alginate (0.04 %) | 10.7 |
| Sodium alginate (0.2 %) | 20.5 |
| Sodium alginate (1.0 %) | 43.1 |
| Sodium alginate (5.0 %) | not less than 4500 |

### Test 2: Pylorus Occlusion Test

### a) On test fluids

### Test fluid A

To 10 mL of triacetin (Wako Pure Chemical Industries, Ltd.), 40 mL of water was added to make a total volume of 50 mL, thereby making test fluid A.

### Test fluid B

Ten milliliters of olive oil (Wako Pure Chemical Industries, Ltd.) and 10 mL of propylene glycol were mixed and water was added to make a total volume of 50 mL, thereby making test fluid B.

### Test fluid C

Half a gram of stearic acid (Wako Pure Chemical Industries. Ltd.) and 20 mL of propylene glycol were mixed and water was added to make a total volume of 50 mL, thereby making test fluid C.

### b) Explanation of the model

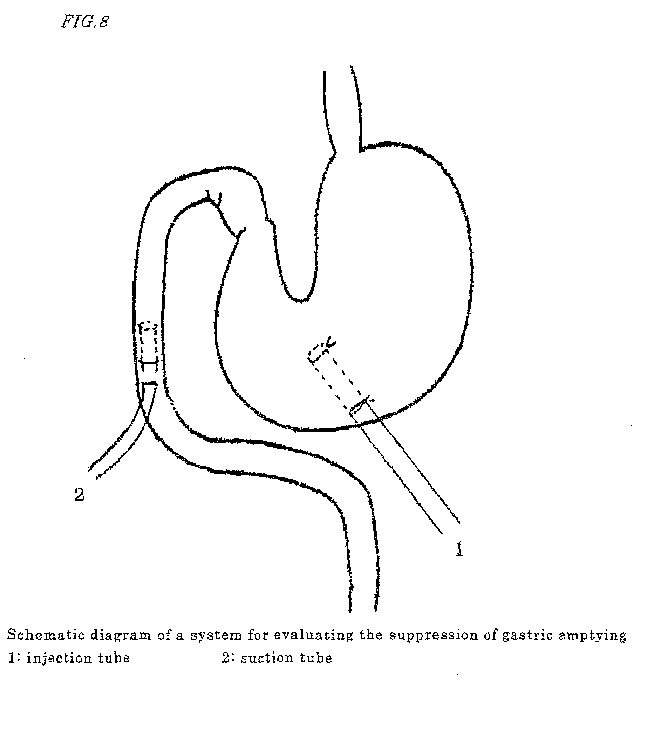

Beagles each weighing about 10 kg were given general anesthesia with an injection of Nembutal (Dainippon Pharmaceutical Co., Ltd.) and following middle incision, tubes were inserted into the body of stomach and the duodenum as shown in FIG. 8 and with each tube being passed subcutaneously through the flank to the back, the dogs received a preliminary invasive operation for an experiment under an unanesthetized condition.

Starting six days after the operation when there were no longer the effects of anesthesia and those of the operative invasion and the general condition had recovered, the study of the sample fluids of interest was started. To be more specific, it is well known that the gastric emptying capability is suppressed under an anesthetized condition, so in the Example under consideration, in order to eliminate that effect and also to determine data for a more normal state, an experiment was conducted under a chronic unanesthetized condition.

### c) Test method

To each test fluid, 5 mg of Evans blue (Wako Pure Chemical Industries, Ltd.) was added amd 50 mL of the mixture was injected into the stomach of each beagle through the tube indicated by 1 in FIG. 8. Similarly, a tube was placed in the duodenum and the test fluid emptied from the stomach was recovered through the tube indicated by 2 in FIG. 8. At given time intervals, the emptied test fluid was recovered by suction (for one minute) through the tube left in place in the duodenum and the Evans blue contained in it was measured for absorbance (DU R640 SPECTRO PHOTOMETER, product of Beckman Inc.) at a wavelength of 605 nm, to thereby measure the concentration of Evans blue in the suctioned fluid; the amount of recovery was used to determine the gastric emptying rate.

### d) Results

With physiological saline used as a comparison, the samples under experiment obviously had a pylorus occluding effect and, in particular, triacetin was markedly effective in occluding the pylorus.

### 1) Triacetin

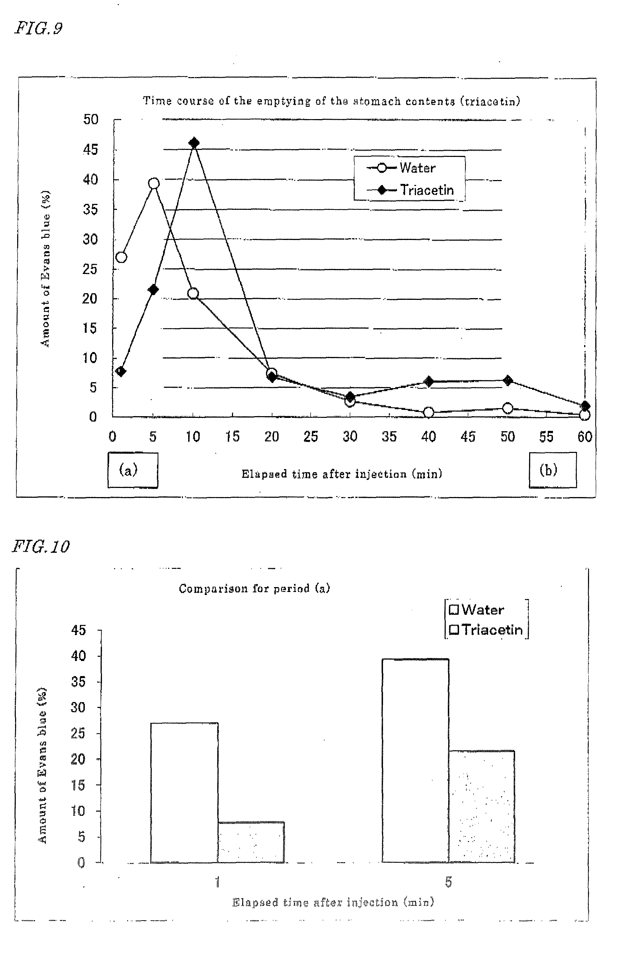

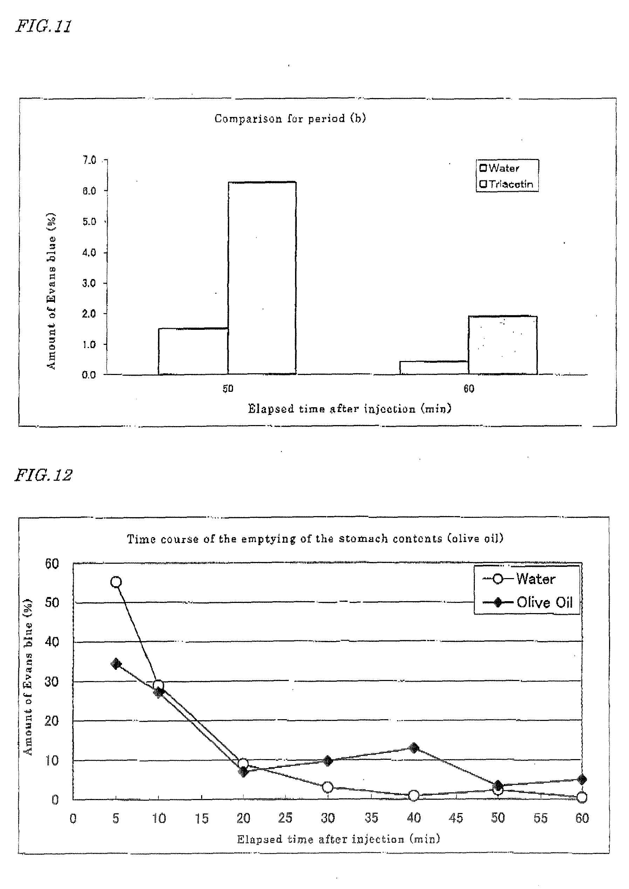

In comparison at the initial stage of administration which is important for the purpose of the present invention, an obvious suppression was recognized as compared with physiological saline, as shown in FIG. 10. As the result, the amount of emptied physiological saline reached a peak at about 5 minutes but triacetin peaked at a later time, about 10-20 minutes, thus fitting the purpose of the present invention. In addition, almost all of the physiological saline had been emptied about 30 minutes after the administration whereas triacetin remained even after 50 minutes and about 5% had been found to be emptied; comparison at this point of time is depicted in FIG. 11, from which one can see an obvious delay in the emptying of triacetin as compared with the physiological saline. It is therefore concluded that the quantitative passage of triacetin is obviously suppressed in comparison with the physiological saline.

### 2) Olive oil

As shown in FIG. 12, the quantitative emptying was such that almost all of the physiological saline had been emptied by 30 minutes after the administration whereas only about 15% of olive oil had been emptied even after 40 minutes, with an obvious suppression of quantitative gastric emptying in comparison with the physiological saline.

### 3) Stearic acid

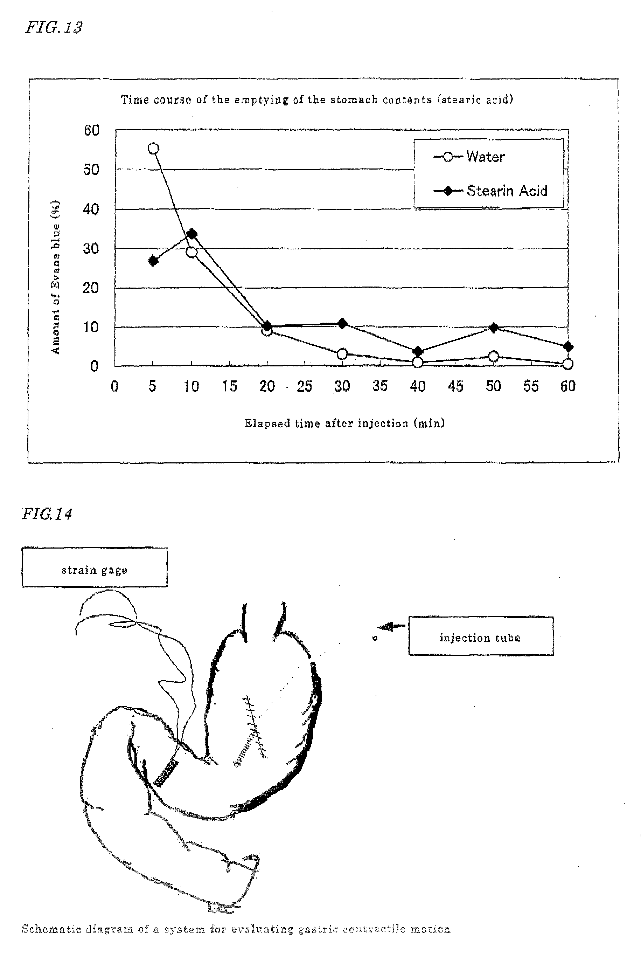

As shown in FIG. 13, the quantitative emptying was such that almost all of the physiological saline had been emptied by 30 minutes after the administration whereas only about 10% of stearic acid had been emptied even after 50 minutes, with an obvious suppression of quantitative gastric emptying in comparison with the physiological saline.

### Test 3: Measurement of Gastric Contractile Motion

### a) On test fluids

### Test fluid D

Two hundred microliters of triacetin (Wako Pure Chemical Industries, Ltd.) was dissolved in distilled water to make a volume of 20 mL, thereby making test fluid D.

### Test fluid B

Four milliliters of olive oil (Wako Pure Chemical Industries, Ltd.) was dissolved in distilled water to make a volume of 20 mL, thereby making test fluid E.

### Control fluid F

Twenty milliliters of distilled water was used as control fluid F.

### b) Explanation of the model

Beagles each weighing about 10 kg were given general anesthesia with an injection of Nembutal (Dainippon Sumitomo Pharma Co.. Ltd.) and following middle incision, a tube for injecting a test fluid into the of stomach and a strain gage (N11-FA-1-120-11-P4-W, NEC Sansei-Instruments Ltd.) were sutured as shown in FIG. 14, with the strain gage being attached to the vestibule which was in the neighborhood of the pyloric band but 2 cm towards the pyloric opening. In order to measure muscular contraction with the strain gage, the submucosa was stretched so that the surface of the mucous membrane would not be incised and the strain gage was buried in the submucosa and sutured thereto, with the wound of incision being closed by a suture.
The tube and the strain gage were subcutaneously guided to the outside of the body from the flank to the back and the dogs received a preliminary invasive operation for an experiment under an unanesthetized condition.
Starting six days after the operation when there were no longer the effects of anesthesia and those of the operative invasion and the general condition had recovered, the study of the sample fluids of interest was started. To be more specific, it is well known that the gastric emptying capability is suppressed under an anesthetized condition, so in the Example under consideration, in order to eliminate that effect and also to determine data for a more normal state, an experiment was conducted under a chronic unanesthetized condition.
Note that signals from the strain gage were amplified (VC-11, NIHON KOHDEN) and then AD converted, with the resulting digital data being analyzed with origin Version 6.5

### (OriginLab Corporation).

### c) Test method

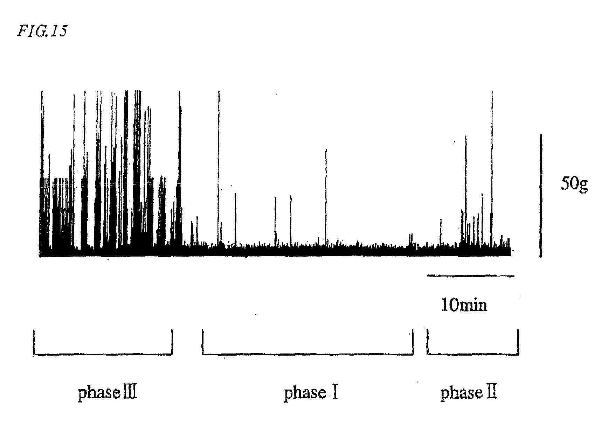

In order to study the effect of triacetin on the contractile motion of the stomach, beagles were placed on a saddle-shaped holder until they were well accustomed to it. Thereafter, the beagles were verified to be in fasting gastric motion by means of the strain gage installed on their muscular tissue (FIG. 15). Phase I corresponding to the rest period was found to occur in the center of FIG. 15, phase II corresponding to an irregular contractile wave on the right side of FIG. 15, and phase III corresponding to a regular strong contractile wave on the left side of the figure. To add the test fluid, it was slowly injected into the stomach through the gastric tube over a period of about one minute in the contraction period of phase III and the occurrence of any reaction for contraction was observed. Note that the beagles on the experiment were allowed to fast, except for water, for at least 15 hours before the start of experiment.

### d) Test results

As the result of comparison with sample fluid E and control fluid F, the Example under consideration showed an obvious suppression of the contractile motion of the stomach by triacetin.

### 1) Triacetin

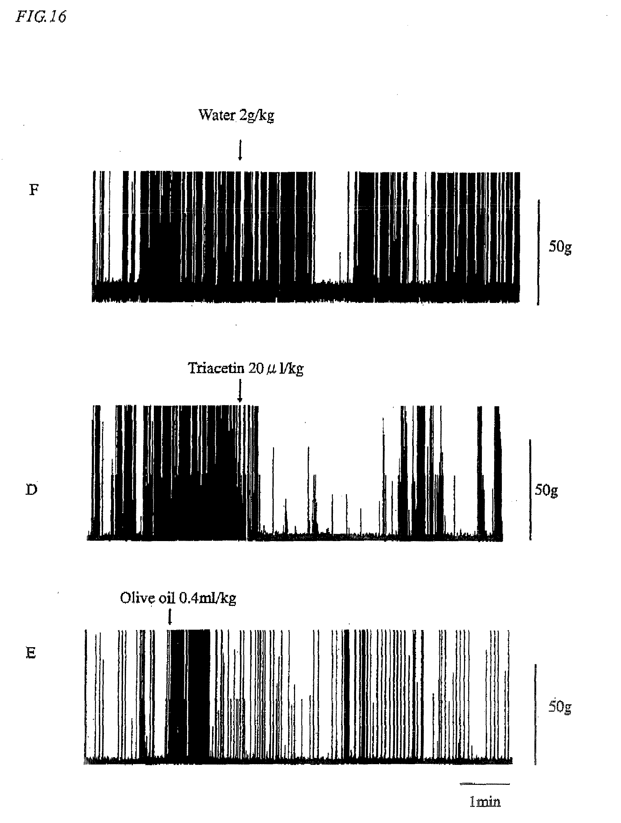

The injection of triacetin (20 µL/kg) decreased both the amplitude and frequency of contractions that had been found to occur before the injection, to thereby suppress the contractile motion (FIG. 16). This change was found to occur immediately after the injection and continued for about three minutes.

### 2) Olive oil

The injection of olive oil (0.4 mL/kg) caused no in the amplitude and frequency of contractions that had been found to occur before the injection (FIG. 16).

### 3) Distilled water

The injection of distilled water (2 g/kg) caused no change in the amplitude and frequency of contractions that had been found to occur before the injection (FIG. 16).

### INDUSTRIAL APPLICABILITY

By incorporating the biocompatible, gastric motility suppressing component such as triacetin in the auxiliary agent for examination, the head and tail of the pancreas the images of which are particularly difficult to acquire can be easily imaged. What is more, by means of observation through the stomach, it also becomes possible to obtain image information from new angles for the edge of the outward area of the left lobe of the liver, the left side of an adrenal, the superior pole and the area inward of the left kidney, and the upper portion of the spleen (under the dome of the left diaphragm). As a further advantage, the mental and physical burdens that may be imposed on the person under examination by treatments such as preliminary ones that precede radiographic or endoscopic examination of the stomach can be reduced.

In addition, triacetin, aside from its use as the above-mentioned auxiliary agent for examination, can be extensively used as a gastric motility suppressor.

## Claims

1. An auxiliary agent for ultrasonic diagnostic examination capable of transmitting ultrasonic wave to an organ, **characterized by** containing at least one biocompatible, gastric motility suppressing component.

2. The auxiliary agent for examination according to claim 1, which contains triacetin as the gastric motility suppressing component.

3. A kit comprising an auxiliary agent for examination capable of transmitting ultrasonic wave to an organ, **characterized by** containing at least one biocompatible, gastric motility suppressing component, an instruction manual for administering the auxiliary agent for examination, and packaging supplies.

4. The kit according to claim 3, which contains triacetin as the gastric motility suppressing component.

5. A gastric motility suppressor containing triacetin as an active ingredient.

6. Use of triacetin in the manufacture of pharmaceuticals for suppressing gastric motility.

7. A method of suppressing gastric motility comprising administering an effective amount of triacetin.

8. A kit comprising a pharmaceutical composition comprising triacetin, an instruction manual describing the method of using the pharmaceutical composition, and packaging supplies.
